# EUROPÄISCHE PATENTANMELDUNG

(11) **EP 1 147 768 A1**
(43) Veröffentlichungstag der Anmeldung: **24.10.2001**
(21) Anmeldenummer: 01107545.4
(22) Anmeldetag: 27.03.2001
(51) Int. Cl.: A61K 31/18, A61K 9/70

(54) **Transdermales therapeutisches System zur Abgabe von Dofetilid**

(30) Priorität: 18.04.2000 DE 10019067
(71) Anmelder: LTS Lohmann Therapie-Systeme AG, 56626 Andernach (DE)
(72) Erfinder: Selzer, Torsten, Dr., 56564 Neuwied (DE)
(74) Vertreter: Schmidt, Werner (DE)

(57) **Zusammenfassung**

Die Erfindung bezieht sich auf ein Dofetilid enthaltendes transdermales therapeutisches System in Pflasterform mit einer wirkstoffundurchlässigen Rückschicht, einem damit verbundenen Wirkstoffreservoir, einer haftklebenden Schicht und einer vor der Applikation ablösbaren Schutzschicht.

Die Erfindung bezieht sich ferner auf die Verwendung derartiger transdermaler therapeutischer Systeme zur medizinischen Anwendung bei verschiedenen Indikationen.

## Beschreibung

Die Erfindung betrifft ein transdermales therapeutisches System, das die Abgabe von Dofetilid (4'-(2-Methyl-4-(methylsulfonylamino)-phenethylaminoethoxy)-methansulfonanilid) an und durch die Haut ermöglicht. Dofetilid hat die Formel

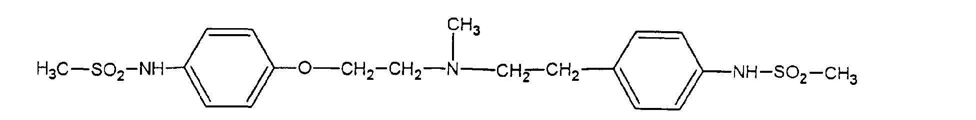

Die Erfindung betrifft ferner die Verwendung eines solchen Systems bei den verschiedensten medizinischen Indikationen.

### Hintergrund der Erfindung

Transdermale therapeutische Systeme (TTS) sind Darreichungsformen, die auf die Haut appliziert werden und die einen Arzneistoff systemisch verfügbar machen sollen. TTS können den therapeutischen Wert einer Arzneistoffverabreichung erhöhen, indem eine konstante Abgabe des Arzneistoffes über einen verlängerten Zeitraum in das Blutgefäßsystem gewährleistet ist. Probleme wie gastrointestinale Intoleranz, niedrige enterale Absorption, Metabolisierung während der ersten Darm-Leber-Passage und hohe Applikationsfrequenz bei niedrigen Halbwertszeiten können damit umgangen werden.

TTS bestehen, nach dem Stand der Technik, aus einer arzneistoffundurchlässigen Trägerschicht, einer arzneistoffhaltigen Reservoirschicht, gegebenenfalls einer Steuermembran, sowie einer Haftklebeschicht zur Befestigung auf der Haut, wobei diese mit der arzneistoffhaltigen Reservoirschicht identisch sein kann, und einer vor Applikation zu entfernenden, ebenfalls arzneistoffundurchlässigen Schutzschicht. Die arzneistoffhaltige Reservoirschicht besteht aus Arzneistoff und Hilfsstoffen, wie Weichmachern, Klebrigmachern, Lösungsvermittlern, Stabilisatoren, Füllstoffen, Trägerstoffen und Permeationsbeschleunigern. Die hierfür in Frage kommenden pharmazeutisch unbedenklichen Substanzen sind dem Fachmann bekannt.

Dofetilid ist ein orales Klasse-III-Antiarrhythmikum, das zur Konversion und Aufrechterhaltung eines normalen Sinusrhythmus bei Patienten mit Vorhofflimmern und -flattern eingesetzt wird. Die Nachteile von Dofetilid ergeben sich aus der Pharmakokinetik: Dofetilid unterliegt bei oraler Gabe einer ausgeprägten Metabolisierung während der ersten Darm-Leber-Passage und besitzt eine geringe Plasmahalbwertszeit.

### Beschreibung der Erfindung

Aufgabe der vorliegenden Erfindung war es, eine Darreichungsform für den Wirkstoff Dofetilid bereitzustellen, die die genannten Nachteile umgeht.

Die Aufgabe wird gelöst durch eine transdermale Darreichungsform in Pflasterform, welche einen genügend hohen Wirkstoff-Flux in vivo ermöglicht und welche mittels gängiger Herstellverfahren kostengünstig produziert werden kann. Aufgrund seiner Eigenschaften, nämlich des hohen Schmelzpunkts (ca. 150 °C) und Molekulargewichts (442g/mol), einem Verteilungskoeffizienten von ca. 1 und der beiden vorhandenen Sulfonanilidgruppen, die zu Wasserstoffbrückenbildung neigen, was erfahrungsgemäß eine transdermale Permeation erschwert, war es nicht zu erwarten, daß Dofetilid in befriedigender Weise in Form von transdermalen Systemen angewendet werden könnte.

Gegenstand der Erfindung ist also ein Dofetilid enthaltendes transdermales therapeutisches System in Pflasterform mit einer wirkstoffundurchlässigen Rückschicht, einem damit verbundenen Wirkstoffreservoir, einer haftfklebenden Schicht und einer vor der Applikation ablösbaren Schutzschicht.

Die Vorteile ergeben sich, wie oben beschrieben, aufgrund der konstanten Abgabe des Wirkstoffes über den Applikationszeitraum. Somit wird die rasche Elimination von Dofetilid durch ständiges Nachliefern aus dem transdermalen System kompensiert. Außerdem wird das Problem der geringen peroralen Verfügbarkeit umgangen.

Die erfindungsgemäßen TTS können sowohl in Form von Matrixsystemen als auch in Form von Membransystemen eingesetzt werden. Der Begriff "Matrixsysteme" schließt nicht nur solche Systeme ein, in denen der Wirkstoff in einer Kunstharz- bzw. Kunststoffmatrix gelöst ist und aus dieser abgegeben wird, sondern auch solche, in denen der Wirkstoff an einem Fasermaterial, wie einem Baumwollgewebe oder -vlies adsorbiert ist. Dabei ist es, insbesondere für Matrixsysteme, unerheblich, welche Polymere, Harze und evtl. weitere Zusatzstoffe eingesetzt werden, sofern die mit der Haut in Berührung kommenden Stoffe hautverträglich sind und die Formulierung geeignet ist, den Wirkstoff Dofetilid an die Haut abzugeben.

Im einfachsten Fall kann Dofetilid in einer Lösung von Grundpolymeren grob, kolloidal oder molekular dispergiert sein, die Mischung auf eine geeignete Unterlage - in der Regel mit einer Silikonschicht versehene thermoplastische Folie - beschichtet und, nach Abdampfen der Lösemittelanteile, mit einer weiteren Folie abgedeckt werden, welche die spätere Rückseite des TTS darstellt. Durch Stanzen flächiger Gebilde in der gewünschten geometrischen Form werden TTS aus einem solchen Laminat erhalten.

Geeignete Grundpolymere für das erfindungsgemäße System sind für die Matrix und den Haftkleber Polymere auf Basis von Acryl- und/oder Methacrylsäure und deren Estern, Isobutylen, Ethylen-Vinylacetat und/oder natürliche und synthetische Kautschuke wie Styrol-Dien-Copolymere, z. B. Styrol-Butadien-Blockcopolymere, oder Isopren-Blockpolymere, Acrylnitril-Butadien-Kautschuk, Butylkautschuk oder Neoprenkautschuk, oder Heißschmelzkleber. Als Haftkleber kommen auch solche auf Silikonbasis in Betracht. Diese Auflistung ist nicht vollständig, läßt aber die breite Anwendungsfähigkeit des erfindungsgemäßen Prinzips erkennen.

Eine bevorzugte Ausführungsform der Erfindung besteht darin, daß das Dofetilid im TTS in Kombination mit einem Lösungsmittel, vorzugsweise im gelösten Zustand vorliegt, wobei die Formulierung möglichst einen Lösungsvermittler enthalten sollte. Beispiele für Lösungsvermittler sind mehrwertige Alkohole wie 1,2-Propandiol, die verschiedenen Butandiole, Glycerin, Polyethylenglycol 400, ferner Tetrahydrofurfurylalkohol, Diethylenglykolmonoethylether, Diethyltoluamid, Monoisopropylidenglycerin usw.1,2-Propandiol wird bevorzugt. Als vorteilhaft hat sich herausgestellt, daß der Anteil des Lösungsvermittlers, bezogen auf das fertige TTS, zwischen 1 und 50 Gew.-% , vorzugsweise zwischen 5 und 35 Gew.-% beträgt. Einige dieser Lösungsvermittler wie das 1,2-Propandiol können auch als permeationsfördernde Stoffe wirken.

Um einen hohen Flux zu erreichen, hat es sich bei Matrixsystemen als besonders vorteilhaft erwiesen, daß permeationsfördernde Stoffe in einer Menge von 0,1 bis 25 Gew.-%, vorzugsweise zwischen 1 und 10 Gew.-% zugesetzt werden, bezogen auf das Gesamtgewicht der Matrix. Beispiele hierfür sind Fettalkohole wie Decanol, Dodecanol, Fettsäuren wie Ölsäure, Myristinsäure, Polyoxyethylenfettalkoholether. Vorzugsweise werden von diesen Polyoxyethylenlaurylether (Brij®) eingesetzt. Auch Polyoxyethylenfettsäureester und Fettsäureester wie Sorbitanmonolaurat oder Ester von langkettigen Fettsäuren mit Methyl-, Ethyl- oder Isopropylalkohol oder Ester von Fettalkoholen mit Essigsäure oder Milchsäure sowie Stoffe wie Ölsäurediethanolamin kommen in Betracht.

Die erfindungsgemäßen TTS können auch einen schichtförmigen Aufbau mit zwei oder mehreren Matrixschichten aufweisen, die z. B. Polymerbestandteile aus der Gruppe der substituierten Cellulosen enthalten, vorzugsweise der Methyl- und Ethylcellulosen. Dabei können die einzelnen Matrixschichten unterschiedliche Konzentrationen an Wirkstoff, permeationsfördernden Mitteln und Lösungsvermittler enthalten. Je nach dem beabsichtigten Anwendungszweck lassen sich die Konzentrationen in diesen Schichten so differenzieren, daß sie von der inneren Schicht zu der Schicht an der Hautseite kleiner oder größer werden, je nachdem, ob eine besondere Langzeitwirkung oder eine Initialwirkung angestrebt wird. Ferner können die einzelnen Matrixschichten aus unterschiedlichen Haftklebern bestehen sowie übliche Weichmacher in einer Konzentration bis zu 30 Gew.-%, vorzugsweise 5 bis 20 Gew.-%, aus der Gruppe Kohlenwasserstoffe, Alkohole, Karbonsäuren und ihre Derivate, Ether, Ester oder Amine enthalten. Um eine Steuerung der Freisetzung zu ermöglichen, sofern dies nicht durch andere Mechanismen bewirkt wird, kann das Reservoir auch mit einer Steuermembran versehen werden, welche die Abgabe des Wirkstoffs an die Haut steuert.

Zur Befestigung des transdermalen Systems auf der Haut gibt es ebenfalls verschiedene Möglichkeiten. Z. B. kann die Matrix selbst aus einem Haftkleber bestehen und so die Verbindung zur Haut herstellen. Es ist aber auch denkbar, das TTS bei Systemen mit einer Steuermembran so herzustellen, daß die Befestigung auf der Haut durch einen Klebstoffrand bewirkt wird, der die Fläche, durch die der Wirkstoff an die Haut abgegeben wird, nicht berührt, also mit der Steuermembran in keiner Verbindung steht.

Das erfindungsgemäße TTS enthält außerdem eine wirkstoffundurchlässige Rückschicht sowie eine ebenfalls wirkstoffundurchlässige ablösbare Schutzschicht bzw. Abziehfolie. Als Rückschicht eignen sich vor allem Polyester, die sich durch besondere Festigkeit auszeichnen, wie Polyethylen- und Polybutylenterephthalat, darüber hinaus aber nahezu beliebige andere hautverträgliche Kunststoffe, wie Polyvinylchlorid, Ethylen-Vinylacetat-Copolymere, Polyvinylacetat, Polyethylen, Polypropylen, Polyurethane, Cellulosederivate und viele andere mehr. Im Einzelfall kann die Rückschicht mit einer zusätzlichen Auflage versehen werden, z. B. durch Bedampfung mit Metallen, insbesondere Aluminium. Für die ablösbare Schutzschicht können dieselben Materialien verwendet werden wie für die Rückschicht, vorausgesetzt, daß sie durch eine geeignete Oberflächenbehandlung wie Silikonisierung ablösbar ausgerüstet ist. Es können aber auch andere ablösbare Schutzschichten wie mit Polytetrafluorethylen behandeltes Papier oder Cellophan® (Cellulosehydrat) verwendet werden.

Der Wirkstoff kann auch in einem beutelförmigen Reservoir vorliegen, welches mit einer fließfähigen, z. B. hochviskosen oder halbfesten Kunststoffmatrix oder einer Lösung davon gefüllt ist, die den Wirkstoff enthält. Besonders vorteilhaft ist es, wenn das Wirkstoffreservoir einen Gelbildner enthält. Die der Haut abgewandte Beutelrückseite muß dabei wirkstoffundurchlässig, die der Haut zugewandte Seite wirkstoffdurchlässig sein. Gegebenenfalls kann eine wirkstoffdurchlässige Membran die Steuerung der Wirkstofffreisetzung übernehmen.

Um eine hohe Freisetzungsrate zu erzielen, ist es bevorzugt, eine möglichst hohe Wirkstoffkonzentration in den wirkstoffhaltigen Schichten vorzusehen, wobei allerdings zu beachten ist, daß bei zu hohen Konzentrationen die physikalische Stabilität beeinträchtigt werden kann. Bei den erfindungsgemäßen TTS werden deshalb Wirkstoffkonzentrationen im Bereich von 0,1 bis 50 Gew.-% , insbesondere von 1 bis 10 Gew.-%, bezogen auf die Gesamtmasse der wirkstoffhaltigen Schichten angewandt.

Die erfindungsgemäßen TTS eignen sich zur medizinischen Anwendung bei den verschiedensten Indikationen wie zur Behandlung von Herz-Rhythmusstörungen, Herzinsuffizienz, peripheren Durchblutungsstörungen, erhöhtem Blutdruck und zur Anfallsprophylaxe der Angina pectoris. Sie eignen sich zur Verabreichung von Dofetilid mit einer Wirkungsdauer von etwa einem Tag bis zu sieben Tagen, je nach Gehalt an Wirkstoff und Ausgestaltung des Steuerungsmechanismus.

### Beispiel

Die erfindungsgemäßen TTS können z. B. wie folgt hergestellt werden:
Es werden 50 g Dofetilid sowie 20 g eines geeigneten permeationsfördernden Stoffes (z.B. Brij®30) in 200g 1,2-Propandiol gelöst. Diese Lösung wird mittels einer geeigneten Rührapparatur in den Silikonkleber 4301 der Fa. Dow Corning (USA), gegeben und dispergiert, so daß möglichst eine homogene Flüssig-Flüssig-Dispersion entsteht. Diese Dispersion wird mit einer geeigneten Vorrichtung homogen auf eine Trägerfolie, z. B. aus Polyethylenterephthalat beschichtet. Anschließend wird durch eine kontrollierte Trocknung das Lösungsmittel des Silikonklebers sowie etwaige Anteile des Propandiols entfernt. Das so erhaltene Laminat wird anschließend mit einer weiteren Folie aus Polyethylenterephthalat zukaschiert. Zuletzt werden TTS einer bestimmten Fläche ausgestanzt und in ein geeignetes Packmittel verpackt.

## Patentansprüche

1. Dofetilid enthaltendes transdermales therapeutisches System in Pflasterform, umfassend eine wirkstoffundurchlässige Rückschicht, ein damit verbundenes Wirkstoffreservoir als Matrix- oder Membransystem, eine haftklebende Schicht und eine vor der Applikation ablösbare Schutzschicht, wobei die Wirkstoffkonzentration im Bereich von 0,1 bis 50 Gew.-% liegt, bezogen auf die Gesamtmasse der wirkstoffhaltigen Schichten.

2. Transdermales therapeutisches System nach Anspruch 1, **dadurch gekennzeichnet, daß** das Dofetilid in Kombination mit einem Lösemittel, vorzugsweise einem solchen, das zugleich eine permeationsfördernde Wirkung hat, insbesondere im gelösten Zustand vorliegt.

3. Transdermales therapeutisches System nach Anspruch 2, **dadurch gekennzeichnet, daß** das Lösungsmittel in einer Menge von 1 bis 50, vorzugsweise 5 bis 35 Gew.-% zugegen ist.

4. Transdermales therapeutisches System nach einem oder mehreren der Ansprüche 1 bis 3, **dadurch gekennzeichnet, daß** es als Matrixsystem vorliegt und einen permeationsfördernden Stoff enthält, zweckmäßig in einer Menge von 0,1 bis 25 Gew.-%, vorzugsweise von 1 bis 10 Gew.-%, bezogen auf das Gesamtgewicht der Matrix.

5. Transdermales therapeutisches System nach einem oder mehreren der Ansprüche 1 bis 4, **dadurch gekennzeichnet, daß** die Wirkstoffkonzentration im Bereich von 0,1 bis 10 Gew.-% liegt.

6. Transdermales therapeutisches System nach einem oder mehreren der Ansprüche 1 bis 5, **dadurch gekennzeichnet, daß** es einen schichtförmigen Aufbau mit mindestens zwei Polymer-Matrixschichten besitzt, vorzugsweise mit unterschiedlicher Konzentration von Dofetilid.

7. Transdermales therapeutisches System nach einem oder mehreren der Ansprüche 1 bis 6, **dadurch gekennzeichnet, daß** die Grundpolymeren für die Matrix und den Haftkleber Polymere auf Basis von Acryl- und/oder Methacrylsäure und deren Estern, Isobutylen, Ethylen-Vinylacetat sind oder Styrol-Butadien-Blockcopolymere oder Isopren-Blockpolymere und/oder daß die Haftkleber Stoffe auf Siliconbasis sind.

8. Verwendung des transdermalen therapeutischen Systems nach einem oder mehreren der Ansprüche 1 bis 7 zur Behandlung von Herz-Rhythmusstörungen und/oder von Herzinsuffizienz und/oder zur Anfallsprophylaxe der Angina pectoris.

9. Verwendung des transdermalen therapeutischen Systems nach einem oder mehreren der Ansprüche 1 bis 7 zur Behandlung von peripheren Durchblutungsstörungen.

10. Verwendung des transdermalen therapeutischen Systems nach einem oder mehreren der Ansprüche 1 bis 7 zur Behandlung von erhöhtem Blutdruck.
